# EUROPÄISCHE PATENTSCHRIFT

(11) **EP 0 979 408 B1**
(45) Veröffentlichungstag und Bekanntmachung des Hinweises auf die Patenterteilung: **06.10.2004**
(21) Anmeldenummer: 98916612.9
(22) Anmeldetag: 20.04.1998
(51) Int. Cl.: G01N 33/543, G01N 21/77

(54) **CLUSTER VERSTÄRKTER OPTISCHER SENSOR**
REINFORCED CLUSTER OPTICAL SENSORS
DETECTEUR OPTIQUE RENFORCE PAR DES CLUSTERS

(30) Priorität: 22.04.1997 AT 68097; 30.09.1997 AT 165697; 30.09.1997 AT 165597
(43) Veröffentlichungstag der Anmeldung: 16.02.2000
(73) Patentinhaber: SCHALKHAMMER, Thomas, 3072 Kasten Nr. 105 (AT); PITTNER, Fritz, 1230 Wien (AT); Bauer, Georg, 4070 Eferding (AT)
(72) Erfinder: SCHALKHAMMER, Thomas, A-3072 Kasten 105 (AT); PITTNER, Fritz, A-1230 Wien (AT); BAUER, Georg, A-4070 Eferding (AT)
(74) Vertreter: Babeluk, Michael, Dipl.-Ing.
(86) Internationale Anmeldenummer: PCT/AT1998/000101
(87) Internationale Veröffentlichungsnummer: WO 1998/048275

(56) Entgegenhaltungen:
- EP-A- 0 300 990
- EP-A- 0 677 738
- EP-A- 0 702 228
- WO-A-90/05295
- WO-A-95/15496
- US-A- 5 507 936

## Beschreibung

Die Erfindung bezieht sich auf ein neuartiges Meßprinzip zum Aufbau von Sensoren und zur Verwendung in der Bioinformatik. Die Technologie beruht dabei auf einem neuartigen plasmonoptischen Meßsystem unter der Verwendung von Clustern, mit welchem insbesondere Nukleinsäuren, Proteine und deren Liganden erfaßt werden können. Die genannten Analyte induzieren dabei die Bindung oder Abtrennung von metallischen Clustern, die in einem bestimmten Abstand zu einer reflektierenden, vorzugsweise elektronenleitenden Oberfläche, gebunden werden bzw. wurden. Die Bindung oder Abtrennung wird durch die Resonanzverstärkung der Cluster, bei der die Cluster mit ihren Spiegeldipolen wechselwirken, in ein leicht meßbares optisches Signal umgewandelt. Es besteht heute großer Bedarf an raschen, einfachen und billigen Testverfahren, in der medizinischen Diagnostik sowie der Lebensmittel- und der Umweltanalytik. Dabei werden immer größere Anforderungen an Empfindlichkeit, Selektivität und Verläßlichkeit bei maximaler Einfachheit des Meßvorgangs gestellt. Die Erfindung zielt darauf ab, durch einen neuartigen Meßaufbau, grundlegende technische Einschränkungen etablierter Analyseverfahren zu beseitigen. Aufbauend auf dieser Technologie können rasche und sichere Schnelltests für Klinik und Labor verwirklicht werden. Als primäre Anwendungsbereiche können z.B. die Diagnose von Harnwegsinfekten, Allergen-Screening, die Quantifizierung von Bakterienkontaminationen in Lebensmitteln oder die Messung der Blutglukose genannt werden.

Die mit Bezugszeichen versehenen Teile des erfindungsgemäßen Aufbaus sind wie folgt zuzuordnen: 1 = Trägermaterial, 2 = reflektierende Schicht (vorzugsweise elektronenleitende Metall oder Clusterschicht), 3 = 0-500 nm Abstandsschicht, 4 = nanometrische, nichtleitende Partikel, 5 = chemisch reaktiver Oberflächenanker, 6 Linker (z.B. DNA, Proteine, ...) 7 = Cluster, 8 = Analyt, 9 = gespaltener Linker (z.B. von einem Analyt gespalten), 10 = nicht gespaltener Linker, 11 = nach Anlagerung eines katalytisch aktiven Analyten, 12 = Sensor Aufbau nach der Spaltung des Linkers, 13 = Abdissoziation des Clusters mit einem darin verankerten Teil des Linkers, 14 = Freie gespaltene Cluster-Linker Konjugate, 15 = Elektroden auf oder nahe dem Chip oder Magnet, 16 = Analyt bindendes Molekül z.B. DNA, Protein, ...., 17 Analytanalog.

Der Sensor besteht aus einer Metallschicht auf einem Trägermaterial, einer inerten Abstandsschicht, z.B. mittels Photolackschleuder oder Aufdampfen aufgebracht, auf der vereinzelt mit Clustern gekoppelte Linkermoleküle gebunden sind. Der Durchmesser der Cluster wird vorzugsweise kleiner als 40 nm gewählt. Wenn der Analyt mit dem Linker interagiert, induziert er eine Änderung der Belegungsdichte der Clusterschicht im molekularen Maßstab, oder Änderungen in der räumlichen Anordnung der gebundenen Cluster am Sensor. Dies führt zu den charakteristischen Änderungen der optischen Erscheinung der Sensoroberfläche. Die durch den anormalen optischen Effekt gefärbte Oberfläche wird dabei durch die katalytische oder biorekognitive Wirkung des Analyten oder durch Zusatz einer enzymatischen aktiven Komponenten verändert. Metallische Clusterfilme mit einem mittleren Clusterdurchmesser kleiner als 500 nm (vorzugsweise kleiner als 40 nm, um Multipol-Peaks im Spektrum zu unterdrücken) weisen starke schmalbandige Reflexionsminima auf, deren spektrale Lagen extrem empfindlich von der räumlichen Anordnung insbesondere dem Abstand zur elektronenleitenden Oberfläche abhängen.

Der Sensoraufbau kann selbst geringste Änderungen der Oberflächenbelegung mit Cluster in ein klar erkennbares optisches Signal umwandeln, entweder in eine starke Extinktionsänderung bei einer bestimmten Wellenlänge, oder in eine spektrale Verschiebung des Absorptionsmaximums. Erfindungsgemäß ist es möglich, biorekognitive Bindungsprozesse und die katalytische Aktivität von Proteinen durch die Verwendung von Oberflächen-gebundenen Clustern in ein optisches Signal (= Farbänderung der Sensoroberfläche) umzuwandeln.

Beispielhaft kann die Sensitivität des Meßaufbaus wie folgt berechnet werden: 25 nm große Cluster werden im Raster von 100 Nanometern angeordnet. Bei einer optischen Auflösung von 1/10 mm, entspricht eine Änderung der Signalstärke um 10% 2x10e5 Molekülen. Diese Sensitivität wurde experimentell mit einem Antikörper - Antigen Aufbau nachgewiesen. Die Verwendung katalytisch aktiver Analyte erhöht die Empfindlichkeit nochmals um einige Zehnerpotenzen und erlaubt damit Einzelmoleküldetektion.

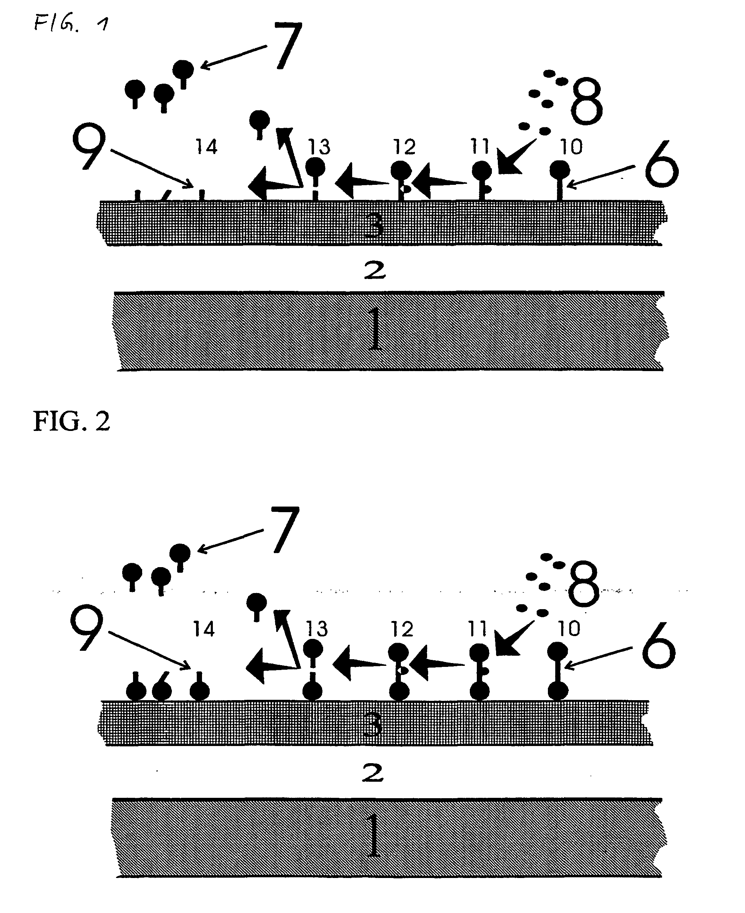

Nanocluster (bevorzugt Silber-, Aluminium- oder Goldcluster) können über sogenannte "biochemische Linker" in definiertem Abstand zur metallisierten Oberfläche gebunden werden. Werden diese Linker durch biochemische Rekognition oder Katalyse durchschnitten oder ihre räumliche Anordnung verändert, so führt das zu einem detektierbaren Signal. Erfindungsgemäß werden z.B. Oligonukleotide als Linker verwendet, welche durch den Analyten geschnitten werden ( z.B. Restriktionsenzyme aus Mikroorganismen) (siehe Fig. 1 und 2). Viele pathogene Mikroorganismen exprimieren spezifische Restriktions-Endonukleasen und können daher mit Hilfe des neuen Einwegmeßsystems rasch, ohne aufwendige apparative Ausführung, in der Arztpraxis oder im Labor, nachgewiesen werden. Dies ermöglicht z.B. die Differentialdiagnose von Harnwegsinfekten durch direkten E. Coli Nachweis (Erreger von 60 % aller Harnwegsinfekte). Ebenso kann eine schnelle und verläßliche Screening-Methode für bakterielle Kontamination in Lebensmitteln aufgebaut werden.

Die neuartige Technologie basiert auf verstärkten Cluster Plasmonen, die in einer sehr einfachen und reproduzierbaren Weise die Aktivität von chemisch reaktiven Spezies in ein optisches Signal umwandeln.

Der Aufbau des Sensors besteht im Wesentlichen darin, daß
1. im Abstand von weniger als 1 µm zu
2. einer reflektierenden, vorzugsweise elektronenleitenden Oberfläche,
3. Linker immobilisiert werden, an die
4. direkt oder indirekt elektrisch leitende Cluster gebunden sind.

Metallische Cluster können somit z.B. auf die Oberfläche eines inerten (nicht reaktiven) Polymers angelagert werden und können auf der Polymeroberfläche über biochemische Linker in definiertem Abstand zur Metalloberfläche angeordnet werden. Die Linker können entweder geschnitten werden, oder ihre räumliche Anordnung kann durch biochemische Rekognition oder Katalyse geändert werden, was sodann beides zu einem optischen detektierbaren Signal führt. Für ein DNA/RNA-Testsystem können Oligonukleotide als Linker eingesetzt werden, die daraufhin durch Restriktionsenzyme geschnitten werden.

Die vorliegende Erfindung unterscheidet sich vom Gegenstand der Patentanmeldung "Optochemischer Sensor sowie Verfahren zu seiner Herstellung", EP-A-0 677 738 durch grundlegende strukturelle Merkmale: Die hier angemeldete Aufbau beinhaltet keine reaktive Matrix, die vorzugsweise Volumen Änderungen ausführen soll. Der neuartige Aufbau basiert auf einer Änderungen der Cluster-Belegungsdichte, dabei sind analytwechselwirkende chemische Linkermoleküle in definiertem Abstand zu einer reflektierenden Schicht gebunden.

Die Bezeichnung anormale Eigenschaft eines Metallfilms bezieht sich auf ein starkes Absorptionsmaximum, zumeist im sichtbaren Spektralbereich, welches durch die räumliche Lokalisierung der Leitungsbandelektronen in den Grenzen des nanometrischen Partikels bewirkt wird. Diese räumliche Lokalisierung steht im Gegensatz zur freien Mobilität der Elektronen in einem makroskopischen Stück Metall (die freie Mobilität der Elektronen ist verantwortlich für die starke Reflexion, allgemein metallischer Glanz genannt).

Ein metallischer Cluster in einem definierten Abstand zu einer metallischen Oberfläche interagiert elektrodynamisch mit der benachbarten Metallschicht. Bei einem definierten Abstand der absorbierenden Clusterschicht von der Metalloberfläche kann das elektrische Feld, das von der Metalloberfläche zurückgeworfen wird in der gleichen Phase wie die einfallende elektromagnetische Welle zu liegen kommen. Der daraus resultierende Rückkopplungsmechanismus verstärkt den effektiven Absorptionskoeffizienten der Clusterschicht. Da bei einer gegebenen Schichtdicke der Abstandsschicht die optimale Phasenverstärkung nur von der Frequenz des eingestrahlten Lichtes abhängt, läßt sich das System durch schmale und starke Reflexionsminima definieren. Die Intensität der Absorptionsbande ist in einem weiten Belegungsbereich mit Cluster direkt proportional der Anzahl der Cluster. Jede Reduktion der Anzahl der Cluster durch chemische Abspaltung resultiert daher in einer Verringerung der Absorption des resonanten Systems. Bei hohen Oberflächenbelegungen wird zusätzlich durch Cluster-Cluster Wechselwirkungen eine spektrale Verschiebung beobachtet (siehe Fig. 2).

Die optische Verhalten des Sensors kann durch die sogenannte Stratified Medium Theorie oder die CPS-Theorie (welche von Chance, Prock und Silbey erstmals vorgeschlagen wurde) beschrieben werden. Diese Theorien beruhen entweder auf dem Verhalten eines optischen Dünnfilms oder auf dem Verhalten eines polarisierbaren Partikels nahe einer Metalloberfläche. Die Stratified Medium Theorie kann zur Berechnung jeder Art optischer Dünnfilme verwendet werden. Sie beruht auf der Lösung der Maxwell Gleichungen unter den Randbedingungen. daß Phasengrenzen und Phasendicke der unterschiedlichen Materialien vorgegeben sind. Um die Stratified Medium Theorie anwenden zu können, müssen daher alle optischen Konstanten der vier Schichten (Oberfläche, Abstandsschicht, Linker Schicht und Clusterschicht) bekannt sein. Die optischen Konstanten eines Inselfilms hängen sehr stark von chemischen und physikalischen Parametern ab und müssen daher experimentell bestimmt werden. Um diese Konstanten bestimmen zu können müssen zumindest Reflexions- und Transmissionsspektren der Cluster bekannt sein. Aus theoretischen Berechnungen kann geschlossen werde. daß eine mittlere Massendicke von 3-7 nm ein maximales Signal erwarten läßt. Abhängig von der Anzahl der Linker gebundenen Cluster kann das Reflexionssignal um bis zu drei Größenordnungen variieren. Unter optimierten Anregungs- und Meßbedingungen können sogar einzelne Cluster beobachtet werden. Bedingt durch die partikuläre Struktur des Cluster oder Kolloidfilms gibt es keinerlei Diffusionsbarrieren für Gase oder Flüssigkeiten.

Die Analytkonzentration kann mit hoher Sensitivität durch Betrachtung der Sensoroberfläche ohne Zuhilfenahme technischer Hilfsmittel bestimmt werden. Um den unspezifischen Hintergrund durch die Eigenfärbung der Probe zu reduzieren kann jedoch eine zwei Winkel Messung eingesetzt werden. Während die Absorption von Chromophoren unabhängig vom Winkel der Beobachtung ist verschiebt sich das spektrale Reflexionsminimum stark mit dem Beobachtungswinkel. Daher kann durch einfach Subtraktion beider Signale der Hintergrund durch Matrixeffekte auf einfache Weise eliminierte werden.

Erfindungsgemäß kann ein Sensor zur Messung spezifischer DNA und RNA Sequenzen in der Weise aufgebaut werden, daß nach Hybridisierung der Analyt-DNA/RNA mit dem Linkernukleotid sich eine neue Restriktionsschnittstelle bildet. Nach Inkubation mit einem Restriktionsenzym kann daher die geschnittene DNA/RNA mit den daran gebundenen Clustern durch einen einfachen Waschschritt von der Oberfläche entfernt werden. Durch die Temperaturstabilität aller Komponenten ist eine direkte Kombination mit PCR möglich.

In analoger Weise können auch Restriktionsenzyme nachgewiesen werden, die doppelsträngige Linker schneiden, oder eine HIV Protease, welche einen HIV spezifischen Peptidlinker schneidet. Insbesondere der Nachweis bakterieller Restriktionsenzyme erfordert den Aufschluß von Zellen im Rahmen des Meßvorgangs, die Zellen können dabei aufgebrochen oder permeiert werden, was für die Sensitivität von grundlegender Bedeutung ist. Eine Anwendung dieses Sensors ist im Bereich der molekularbiologischen Forschung zur Bestimmung von Aktivität oder Reinheit von Restriktionsenzymen.

Die folgenden vier Beispiele beschreiben die technische Realisierung des Sensors:

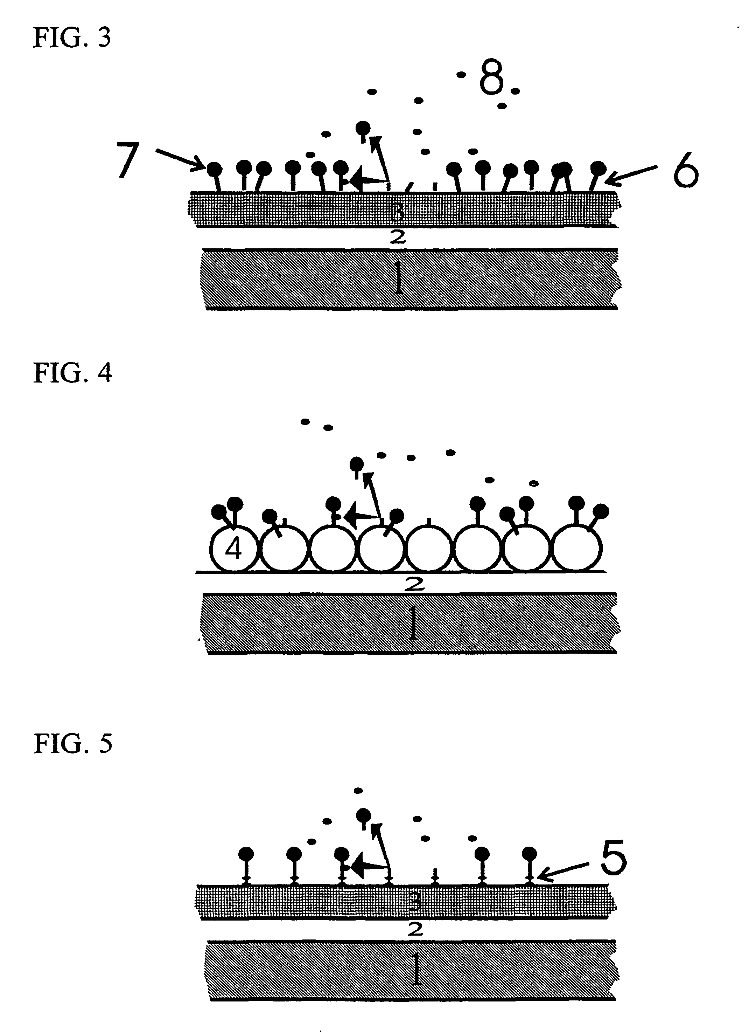

### Beispiel 1 (siehe Fig. 3)

Statistische Kopplung von Linkem nach einem chemischen Standardprotokoll an einen mit einer Photolackschleuder hergestellten inerten nanometrischen Dünnfilm.

### Beispiel 2 (siehe Fig. 4)

Verwendung nanometrischer Partikel insbesondere aus Polystyrol als Distanzschicht. Diese Aufbau ermöglicht homogene Schichtdicken auf unebenen und gekrümmten Oberflächen.

### Beispiel 3 (siehe Fig. 5)

Die Technologie verwendet eine verbesserte Version der Mikrostrukurierung

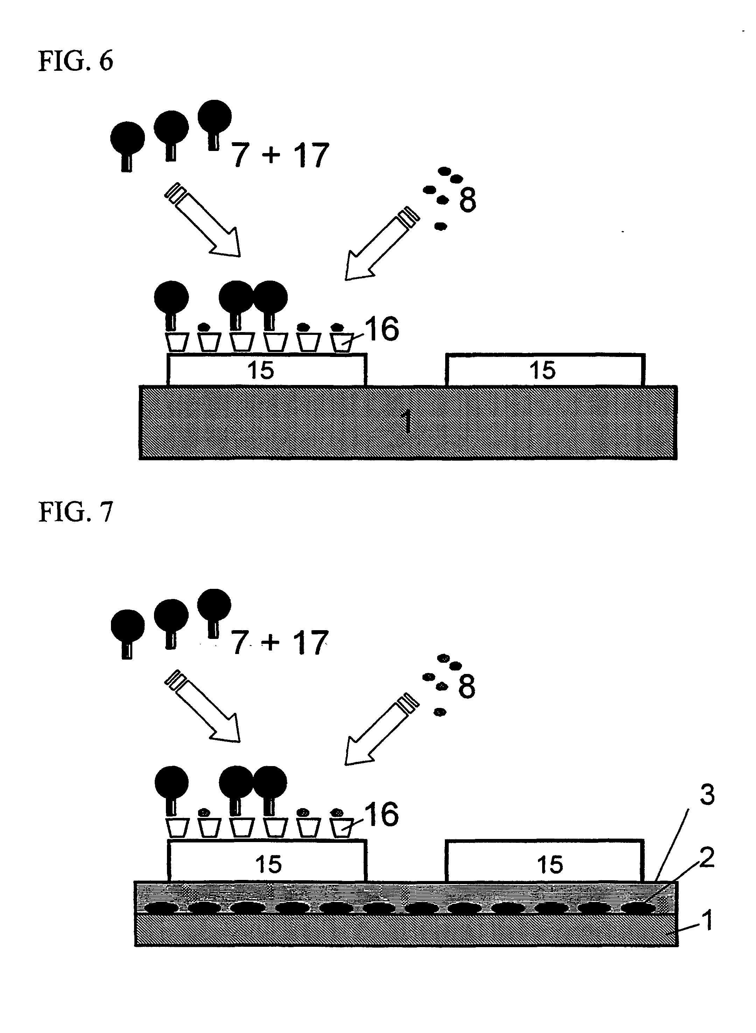

### Beispiel 4 (siehe Fig. 6 und 7)

Diese Technologie verwendet elektrophoretische Bewegung von Clustern.

Beispiel 1: Auf Polyethylenterephtalat welches mit Aluminium metallisiert ist (Widerstand 2Ω) wird eine 6%ige Lösung von Polyhexylmethacrylat mit der Photolackschleuder aufgebracht (4000 rpm, 60 s). Die Oberfläche des Dünnfilms wird mit Sauerstoffplasma chemisch hydroxyliert, carboxyliert und carbonyliert. Mit unspezifischer Adsorption oder mit einem wasserlöslichen Carbodiimid wird daran sodann ein glykosyliertes Protein oder Zuckerderivat gebunden. Sodann wird das tetravalente Concanavalin A an diese Schicht gebunden, wobei eine chemisch reaktive, zuckerbindende Oberfläche entsteht.

Goldkolloide mit einem Durchmesser von 14 nm werden mit einem glykosylierten Protein stabilisiert (z.B. Peroxidase). Aggregation und die unspezifische Adsorption wird durch Zugabe von 0.1% Tween 20 unterdrückt. Nach Zugabe des Analyten erfolgt der Bindungsvorgang unter kompetitiver Reaktion an der Chipoberfläche. Dabei werden die gebundenen Cluster den gewünschten optischen Effekt zeigen, da sie in definiertem Abstand von der reflektierenden Oberfläche festgehalten werden.

Beispiel 2: Nanometrische Partikel auf Polystyrol sind ebenfalls als Abstandsschicht geeignet. Eine durch Bedampfung hergestellte Silberoberfläche wird mit einer 2%igen Lösung von Cystamin (30 Minuten) zur Reaktion gebracht wobei sich ein "Selfassembling Monolayer" mit freien Aminogruppen ausbildet. Carboxylierte Polystyrolkugeln (Durchmesser z.B. 50 nm) könne sodann in einem Zweischrittprotokoll mit wasserlöslichem Carbodiimid an die Aminogruppen gebunden werden. In analoger Weise können carboxylierte Polystyrolkugeln auch an aminosilanisierte Metalloberflächen gebunden werden. Wenn die Bindungsreaktion abgeschlossen ist bildet sich ein dichter, chemisch reaktiver, zweidimensionaler Raster von sphärischen Partikeln aus. Auf den Kunststoffkugeln könne sodann Oligonukleotide mit einem künstlich eingeführten Aminoterminus mit z.B. wasserlöslichem Carbodiimid gebunden werden. Dabei ist die Zugabe von Imidazol nötig, um die Reaktivität des Carbodiimid selektiv auf die terminale Aminofunktion zu reduzieren.

Beispiel 3: Carboxylierte Kugeln von Polystyrol werden auf die in Beispiel 1 beschriebene Polymethacrylatschicht mit Hilfe bifunktioneller aber spaltbarer Vernetzer gebunden. Die überschüssigen reaktiven Gruppen des Polymers werden via Esterbindung inaktiviert. Die Kugeln formen ebenfalls einen zweidimensional geordneten Gitterraster, der nach Spaltung der Vemetzer (z.B. Spaltung von SS-Brücken mit Hilfe eines Reduktionsmittels) einen zweidimensionalen Abdruck von reaktiven SH Gruppen auf der Oberfläche hinterläßt. Die reaktiven SH-Gruppen können sodann als Ankerpunkte für die geordnete Immobilisierung der Linker (z.B. über Iodacetyl-Aktivierung) dienen.

Beispiel 4: Dieser Sensor wird gleichartig aufgebaut wie in Beispiel 1, zusätzlich werden jedoch zumindest zwei Elektroden (zumeist aus Pt, Au, Ag, Pd oder Stahl) am Chip angebracht um Mikroelektrophorese zu ermöglichen. Es erweist sich insbesondere als vorteilhaft die Distanzschicht selbst elektrisch- oder ionenleitfähig auszubilden und als Elektrode zu verwenden. Dabei kann z.B. Indiumzinnoxid aus Plasma aufgedampft werden, oder ein ionenleitendes Polymer eingesetzt werden. Das Anlegen eines elektrophoretischen Signals induziert die Bewegung der elektrisch leitenden Cluster. Dazu kann entweder die lokale Konzentration erhöht werden oder aber ungebundene Cluster entfernt werden. Damit kann die Analysezeit deutlich verringert werden. In gleichartiger Weise können auch nanomagnetische Partikel z.B. Metall-Metalloxid (zumeist Eisen oder Chromoxid) durch (elektro-)magnetische Kräfte bewegt werden.

## Patentansprüche

1. Optischer Sensor mit einer elektromagnetische Wellen reflektierenden Schicht (2), wobei im Abstand von weniger als 1 µm zur reflektierenden Schicht (2) mit einem Analyten (8) wechselwirkende Linker (6) immobilisiert sind, an welche elektrisch leitende Cluster (7) mit einem Durchmesser von weniger als 500 nm gebunden sind, wobei die Zahl der gebundenen Cluster (7) durch eine Interaktion mit dem Analyten (8) veränderbar ist.

2. Optischer Sensor nach Anspruch 1, **dadurch gekennzeichnet, dass** als Linker (6) DNA, RNA, Proteine, Peptide oder ihre Liganden verwendet werden.

3. Optischer Sensor nach Anspruch 1, **dadurch gekennzeichnet, dass** Linker (6) verwendet werden, welche durch den Analyten (8) spaltbar sind.

4. Optischer Sensor nach Anspruch 1, **dadurch gekennzeichnet, dass** die Linker (6) an die Oberfläche einer inerten Abstandsschicht (3) gebunden sind, wobei die Distanz zur reflektierenden Schicht (2) weniger als 500 nm beträgt.

5. Optischer Sensor nach Anspruch 1, **dadurch gekennzeichnet, dass** die reflektierende Schicht (2) aus Metall, einer Metalloberfläche oder aus einer Schicht von Metallclustern besteht.

6. Optischer Sensor nach Anspruch 1, **dadurch gekennzeichnet, dass** als Linker (6) ds-DNA, ds-RNA oder ds-synthetische Analoga verwendet werden, welche durch Restriktionsenzyme spaltbar sind.

7. Optischer Sensor nach Anspruch 1, **dadurch gekennzeichnet, dass** als Linker (6) ss-DNA, ss-RNA oder ss-synthetische Analoga verwendet werden, welche mit dem Analyten (8) hybridisieren.

8. Optischer Sensor nach Anspruch 7, **dadurch gekennzeichnet, dass** der gebildete Doppelstrang durch ein Restriktionsenzym spaltbar ist, das die Einzelstränge nicht spaltet.

9. Optischer Sensor nach Anspruch 1, **dadurch gekennzeichnet, dass** die Cluster durch chemische Synthese erzeugt werden.

10. Optischer Sensor nach Anspruch 1, **dadurch gekennzeichnet, dass** die Cluster (7) aus der Gruppe der Metalle Silber, Gold, Aluminium, Kupfer, Indium bestehen oder aus Metallen und Legierungen welche keine störenden Interbandübergänge besitzen.

11. Optischer Sensor nach Anspruch 1, **dadurch gekennzeichnet, dass** der Abstand zwischen reflektierenden Schicht (2) und den Linkem (6) durch die Anlagerung von nanometrischen Partikeln geeigneter Größe erzeugt wird.

12. Optischer Sensor nach Anspruch 1, **dadurch gekennzeichnet, dass** eine regelmäßige Anordnung von Linkern (6) durch Anlagerung nanometrischer Partikel an die reflektierende Schicht (2) erzeugt wird.

13. Optischer Sensor nach Anspruch 1, **dadurch gekennzeichnet, dass** eine regelmäßige Anordnung von Linkern (6) durch chemische Abdrücke nach Anlagerung nanometrischer Partikel erzeugt wird.

14. Optischer Sensor nach Anspruch 1, **dadurch gekennzeichnet, dass** als Linker (6) Proteine oder Peptide verwendet werden, die durch proteolytische Enzyme spaltbar sind.

15. Optischer Sensor nach Anspruch 1, **dadurch gekennzeichnet, dass** als Linker (6) Antigen-Antikörper oder Rezeptor-Ligand-Konjugate verwendet werden, wobei der Analyt (8) eine der beiden Komponente verdrängt.

16. Optischer Sensor nach Anspruch 1, **dadurch gekennzeichnet, dass** das Sensorsignal durch eine zweite Population von Clustern verstärkt wird.

17. Optischer Sensor nach Anspruch 1, **dadurch gekennzeichnet, dass** der Sensor zusätzlich Elektroden (15) zur elektrophoretischen Bewegung oder Magnete zur magnetischen Bewegung der Cluster (7)aufweist.

18. Verwendung des optischen Sensors nach einem der Ansprüche 1 bis 17 zur Messung von Hormonen, Viren, Bakterien, Proteinen, Peptiden, natürliche und synthetische toxische Substanzen, Pestiziden, DNA und RNA.

19. Verwendung des optischen Sensors nach einem der Ansprüche 1 bis 17 um ein einmal chemisch erzeugtes zweidimensionales Muster wiederholt ablesen zu können.

## Claims

1. Optical sensor with an electromagnetic waves reflecting layer (2), wherein at a distance of less than 1 µm to said reflecting layer (2) linkers (6) interacting with an analyte (8) are immobilized, onto which linkers (6) electrically conducting clusters (7) with a diameter smaller than 500 nm are bound, the number of bound clusters (7) being subject to change by interaction with the analyte (8).

2. Optical sensor according to claim 1, wherein DNA, RNA, proteins, peptides, or ligands thereof are used as linkers (6).

3. Optical sensor according to claim 1, wherein ligands (6) are used which are cleavable by the analyte (8).

4. Optical sensor according to claim 1, wherein the ligands (6) are attached to the surface of an inert distance layer (3), the distance to the reflecting layer (2) being less than 500 nm.

5. Optical sensor according to claim 1, wherein the reflecting layer (2) is made of metal, a metal surface, or a layer of metal clusters.

6. Optical sensor according to claim 1, wherein the linkers (6) comprise ds-DNA, ds-RNA, or ds-synthetic analogues which can be cleaved by restriction enzymes.

7. Optical sensor according to claim 1, wherein the linkers (6) comprise ss-DNA, ss-RNA, or ss-synthetic analogues which will hybridize with the analyte (8).

8. Optical sensor according to claim 7, wherein the double strand formed can be cleaved by a restriction enzyme that will not cleave the single strands.

9. Optical sensor according to claim 1, wherein the clusters are produced by chemical synthesis.

10. Optical sensor according to claim 1, wherein the clusters (7) are selected from the group consisting of silver, gold, aluminium, copper, indium, or any other metals or alloys that have no interfering interband transitions.

11. Optical sensor according to claim 1, wherein the distance between the reflecting layer (2) and the linkers (6) is established by attaching nanometric particles of suitable size.

12. Optical sensor according to claim 1, wherein an array of linkers (6) is formed by attaching nanometric particles to the reflecting layer (2).

13. Optical sensor according to claim 1, wherein an array of linkers (6) is produced by chemical imprints after the attachment of nanometric particles.

14. Optical sensor according to claim 1, wherein the linkers (6) comprise proteins or peptides which can be cleaved by proteolytic enzymes.

15. Optical sensor according to claim 1, wherein the linkers (6) comprise antibody-antigen or receptor-ligand conjugates, in which one of the partners can be replaced by the analyte (8).

16. Optical sensor according to claim 1, wherein the sensor signal is enhanced by a second population of clusters.

17. Optical sensor according to claim 1, wherein the sensor is further provided with electrodes (15) for electrophoretic movement or magnets for magnetic movement of the clusters (7).

18. Use of the optical sensor according to any of claims 1 to 17 for testing hormones, viruses, bacteria, proteins, peptides, natural and synthetic toxic substances, pesticides, DNA and RNA.

19. Use of the optical sensor according to any of claims 1 to 17 for repeated readings of a two-dimensional pattern once it is chemically generated.

## Revendications

1. Détecteur optique comportant une couche (2) réfléchissant les ondes électromagnétiques, selon lequel, à une distance inférieure à 1 µm par rapport à la couche réfléchissante (2), sont immobilisés des linkers (6) coopérant avec un analyte (8), ces linkers étant liés à des clusters (7) conducteurs électriques, d'un diamètre inférieur à 500 nm, et le nombre de clusters (7), liés, peut être modifié par interaction avec l'analyte (8).

2. Détecteur optique selon la revendication 1,
**caractérisé en ce que**
comme linker (6) on utilise l'ADN, l'ARN, des protéines, des peptides ou leurs ligands.

3. Détecteur optique selon la revendication 1,
**caractérisé en ce qu'**
on utilise des linkers (6) qui peuvent être divisés par les analytes (8).

4. Détecteur optique selon la revendication 1,
**caractérisé en ce qu'**
on fixe les linkers (6) à la surface d'une couche d'écartement (3), inerte, et la distance par rapport à la couche réfléchissante (2) est inférieure à 500 nm.

5. Détecteur optique selon la revendication 1,
**caractérisé en ce que**
la couche réfléchissante (2) est en métal, en une surface métallique ou en une couche de clusters métalliques.

6. Détecteur optique selon la revendication 1,
**caractérisé en ce que**
comme linker, on utilise des ds-ADN, ds-ARN ou des ds-analogues de synthèse qui peuvent être décomposés par des enzymes de restriction.

7. Détecteur optique selon la revendication 1,
**caractérisé en ce que**
comme linkers (6) on utilise des ss-ADN, ss-ARN ou des ss-analogues de synthèse et qui peuvent être hybridés avec l'analyte (8).

8. Détecteur optique selon la revendication 7,
**caractérisé en ce que**
la double ligne formée peut être divisée par un enzyme de restriction qui ne divise pas les lignes simples (cordons simples).

9. Détecteur optique selon la revendication 1,
**caractérisé en ce que**
les clusters sont formés par synthèse chimique.

10. Détecteur optique selon la revendication 1,
**caractérisé en ce que**
les clusters (7) sont formés du groupe des métaux argent, or, aluminium, cuivre, indium ou des métaux et des alliages n'ayant pas de jonction interbande perturbatrice.

11. Détecteur optique selon la revendication 1,
**caractérisé en ce que**
la distance entre la couche réfléchissante (2) et les linkers (6) est obtenue par l'insertion de particules nanométriques de dimensions appropriées.

12. Détecteur optique selon la revendication 1,
**caractérisé par**
une répartition régulière de linkers (6) par la répartition de particules nanométriques sur la couche réfléchissante (2).

13. Détecteur optique selon la revendication 1,
**caractérisé par**
une répartition régulière de linkers (6) par des impressions chimiques après stockage de particules nanométriques.

14. Détecteur optique selon la revendication 1,
**caractérisé en ce que**
les linkers (6) sont des protéines ou des peptides qui peuvent être décomposés par des enzymes protéolytiques.

15. Détecteur optique selon la revendication 1,
**caractérisé en ce que**
les linkers (6) sont des antigènes-anticorps ou des récepteurs-ligands-conjugués, et l'analyte (8) est refoulé par l'un des deux composants.

16. Détecteur optique selon la revendication 1,
**caractérisé en ce que**
le signal de détecteur est amplifié par une seconde population de clusters.

17. Détecteur optique selon la revendication 1,
**caractérisé en ce que**
le capteur comporte des électrodes supplémentaires (15) pour créer des déplacements par électrophorèse ou des aimants pour déplacer de manière magnétique le cluster (7).

18. Application d'un détecteur optique selon l'une des revendications 1 à 17 pour mesurer des hormones, des virus, des bactéries, des protéines, des peptides, des substances toxiques naturelles ou de synthèse, des pesticides, des ADN et ARN.

19. Utilisation de détecteurs optiques selon l'une des revendications 1 à 17, pour pouvoir lire de manière répétée un modèle bidimensionnel généré une seule fois par voie chimique.
